# EUROPEAN PATENT SPECIFICATION

(11) **EP 1 119 378 B1**
(45) Date of publication and mention of the grant of the patent: **28.05.2003**
(21) Application number: 99956437.0
(22) Date of filing: 07.10.1999
(51) Int. Cl.: A61L 15/28, A61L 15/60

(54) **POLYSACCHARIDE BASED ABSORBENT POLYMER MATERIAL**
ABSORBIERENDES POLYMERMATERIAL AUF POLYSACCHARIDBASIS
MATERIAU POLYMERE ABSORBANT A BASE DE POLYSACCHARIDE

(30) Priority: 09.10.1998 SE 9803448
(43) Date of publication of application: 01.08.2001
(73) Proprietor: SCA Hygiene Products AB, 405 03 Göteborg (SE)
(72) Inventor: ANNERGREN, Jeanette, S-435 35 Mölnycke (SE); LUNDSTRÖM, Kerstin, S-441 95 Alingsas (SE); ÖSTMAN, Asa, S-412 59 Göteborg (SE)
(74) Representative: Romare, Laila Anette
(86) International application number: SE9901793
(87) International publication number: WO00021581

(56) References cited:
- WO-A2-95/31500

## Description

### TECHNICAL AREA:

The invention relates to a method for manufacturing of a highly absorbent, polysaccharide-based absorption material, wherein a water containing solution comprising a starting material in the form of a crosslinkable polysaccharide-based polymer is subjected to crosslinking in order to produce a water-swelled gel.

### BACKGROUND:

For many applications, such as in absorbent articles intended for absorption of body fluids, it has become increasingly more common to use what is known as superabsorbent materials. Superabsorbent materials are polymers which are capable of absorbing liquid in amounts corresponding to several times the weight of the polymer and which upon absorption form an aqueous gel.

The main advantage of using superabsorbent materials in absorbent articles is that the volume of the absorbent articles can be considerably reduced in comparison to the volume of absorbent articles mainly formed from absorbent fibrous materials such as fluffed cellulose pulp, or the like. Another advantage is that superabsorbents, when compared to fibrous absorbents such as, for instance, fluffed cellulose pulp, have a higher capability of retaining liquid under pressure. Such a property is, for instance, advantageous when the absorption material is used in diapers, incontinence guards or sanitary napkins, since absorbed body fluid is retained in the absorbent article and is not squeezed out of the article, for instance when the user is sitting down.

However, a disadvantage with many of the superabsorbent materials presently being used in absorbent articles such as diapers, incontinence protectors or sanitary napkins, is that they are not produced from renewable raw materials. Accordingly, it has been suggested that superabsorbents based on different types of renewable starting materials, such as polysaccharides and, in particular, starch, be used. Unfortunately, the polysaccharide-based superabsorbents which have so far been available exhibit considerably lower absorption capacity than the commonly used polyacrylate-based superabsorbents. Further, the ability of the polysaccharide-based superabsorbents to retain absorbed liquid when the superabsorbent is subjected to compressive forces is low in comparison with polyacrylate-based superabsorbents.

In WO 95/31500, a method for producing absorbent, preferably superabsorbent, foam materials by phase separation and crosslinking of a polymer solution is described. The absorbent materials obtained exist in the form of a crosslinked open-celled polymer foam, which implies that fluid may pass between cells. By means of the described production method, it is also said to be possible to obtain biodegradeable absorbent foam materials. Preferred polymers for producing the absorbent materials which are disclosed in WO 95/31500 are hydroxyethyl cellulose (HEC) and hydroxypropyl cellulose (HPC), which are preferably crosslinked with divinyl sulphone (DVS).

The known absorbent foam materials are relatively expensive to produce and are primarily intended for medical applications, such as controlled release systems or as artificial skin and blood vessels. However, a further possible use for the described foam materials is said to be in reusable diapers or the like. The high production cost does, however, mean that the known foam materials would, in practice, not be contemplated as absorption material for absorbent articles intended for single use only.

For these reasons, there exists a demand for an improved superabsorbent material based on renewable raw materials. Accordingly, the absorption capacity for polysaccharide-based superabsorbents needs to be improved in order to make such superabsorbents an equal alternative with regard to absorbency and when compared to the superabsorbents which are commonly used today. Moreover, there exists a need for a disposable absorbent article comprising an absorbent structure with a superabsorbent material which may be produced using low-cost, readily available, renewable starting materials.

### DESCRIPTION OF THE INVENTION:

The present invention provides a process for the production of superabsorbent materials of the kind mentioned in the introduction and which exhibit improved absorbency as compared to previously known superabsorbent materials of the same type.

The process according to the invention is primarily distinguished in that the at least partially crosslinked, liquid-swollen gel is soaked in a water-miscible organic solvent so that water is partially removed from the gel prior to desiccation of the gel. Preferably, the liquid-swollen gel is soaked in excess methanol.

The invention further provides a highly absorbent polysaccharide-based absorption material which has been produced by soaking an at least partially cross-linked liquid-swollen gel in a water-miscible organic solvent, preferably methanol, prior to desiccation of the gel.

An absorbent structure comprising the highly absorbent polysaccharide-based absorption materials in accordance with the invention is also disclosed.

When preparing the crosslinked, water-swelled gel, a wide range of solvents may be used for the initial solution containing the polysaccharide-based polymer starting material. However, the solution containing the starting material is preferably an aqueous solution.

Surprisingly, it has been shown that by soaking a crosslinked polysaccharide in methanol, a superabsorbent material can be obtained exhibiting superior absorbency when compared to a material of the same composition but which has been produced by conventional methods. The improved absorbency is evident both in a higher absorption capacity and in a greater ability to retain absorbed liquid even when the absorption material is placed under load. The absorbency of a superabsorbent material which has been soaked in methanol prior to drying of the gel is considerably higher than that of a corresponding superabsorbent material where no methanol soaking step has been performed, regardless of whether the absorbed liquid is water or a salt-containing solution such as urine.

The starting material may comprise a polymer blend comprising an electrically charged polysaccharide-based polymer and an electrically uncharged polysaccharide-based polymer. The ratio between the charged polymer and the uncharged polymer is preferably between about 2:1 and about 4:1 and most preferably about 3:1.

An advantage afforded by the invention is that carboxymethyl cellulose (CMC) can be used as a starting material for the production of a superabsorbent material displaying high absorption capacity and good liquid retention. The fact that CMC is produced from wood which is a renewable material source and, further, that it is readily available and comparatively low in cost, makes CMC particularly suitable for use in disposable absorbent articles. Moreover, with regard to biodegradability and compostability, CMC exhibits excellent characteristics.

However, it has been found to be less suitable to use CMC as sole starting material for the production of a superabsorbent material, since CMC tends to form intramolecular crosslinks instead of crosslinks between molecules. An absorption material having particularly good properties may thus be obtained with a starting material comprising a mixture of CMC in the form of its sodium salt (CMCNa) and hydroxyethyl cellulose (HEC). A suitable proportion between the amount of CMCNa and HEC has thereby been found to be between about 2:1 and about 4:1 and preferably about 3:1. At a lower concentration of HEC, the resulting cross-linked gel does not exhibit sufficient gel strength. High concentrations of HEC should be avoided since the swelling capacity and, accordingly, the absorption capacity will be insufficient if the HEC concentration is too high.

Alternatively, it is possible to use combinations of other charged and uncharged polysaccharides. Some further examples of suitable charged polysaccharides are carboxymethyl starch, oxidized starch and oxidized cellulose. Suitable uncharged polysaccharides include, but are not limited to: ethylhydroxyethyl cellulose (EHEC), hydroxypropyl cellulose (HPC), guar gum and hydroxypropyl starch (HPS).

It is further possible to use pectin or alginate as a starting material.

The polymer solution is preferably crosslinked with a crosslinking agent producing covalent crosslinks. Some examples of crosslinking agents of this kind are divinylsulphone (DVS), acetaldehyde, formaldehyde, glutaraldehyde, diglycidyl ether, diisocyanates, dimethyl urea, epichlorohydrin, oxalic acid, phosphoryl chloride, trimetaphosphate, trimethylomelamine and polyacrolein. Naturally, it is also possible to use ionic crosslinking or physical crosslinking such as hydrophobic/hydrophilic interactions, or the like. Crosslinking may alternatively be performed enzymatically or with radiation.

A superabsorbent material of the above-described kind may be readily combined with fibres and can accordingly be mixed with absorbent fibres such as fluffed cellulose pulp, rayon, peat moss, cotton, hemp, flax, nylon, or the like, using any conventional method. Furthermore, the highly absorbent material may be mixed with non-absorbent fibres such as polyethylene, polypropylene, polyester, bicomponent fibres, or the like. Clearly, it is possible to mix different types of fibres in an absorbent fibrous structure in order to achieve an optimal combination of characteristics such as absorbency, liquid retention, shape stability, and resiliency. The fibrous structure may be bonded, for instance by fusing of thermoplastic fibres comprised in the fibrous structure, or by adding a special binding agent. In addition, the fibrous structure may have been subjected to further processing, such as compression, needling, softening, or the like.

The highly absorbent material may, of course, alternatively be placed in a layer in an absorbent body comprising further layers of fibres, nonwoven sheets, tissue paper, or the like. The highly absorbent material may be a self-sustaining layer, or may be applied onto or within a substrate. Some examples of materials which may serve as substrates are tissue sheets, foam materials, nonwoven sheets, fibrous webs, structures having pockets or recesses in which the highly absorbent material is arranged, or the like.

After preparation of the crosslinked aqueous gel, the gel was dried to produce a highly absorbent material for use in absorbent structures. Several desiccation methods exist. The simplest method is air drying at atmospheric pressure which simply means that the swollen hydrogel is left at room conditions (25°C and 50% relative humidity) until completely dry. Alternatively, the gel may be dried using vacuum or by extraction with a solvent, for example acetone.

### BRIEF DESCRIPTION OF FIGURES:

The invention will be described in greater detail in the following, by way of example only, and with reference to the attached Figures, wherein:
- Fig. 1: shows the free swelling capacity and liquid retention for gels which have been treated with different solvents;
- Fig. 2: shows free swelling capacity and liquid retention for air-dried gels produced from mixtures having different CMC:HEC ratios;
- Fig. 3: shows free swelling capacity and liquid retention for air-dried gels produced from guar gum and which have been treated with different solvents;

### DETAILED DESCRIPTION OF METHODS AND EXAMPLES:

### Gel Preparation

The hydrogels which were used in the following examples, were obtained by crosslinking mixtures of CMCNa and HEC, using DVS as crosslinking agent. The reason for choosing DVS as crosslinking agent is that DVS provides a reliable and reproducible crosslinking result. Thus, DVS is well suited for the production of crosslinked materials for use in comparative work. However, the invention shall not in any way be regarded as being restricted to the use of DVS as crosslinking agent. Accordingly, and as mentioned above, any suitable crosslinking agent or crosslinking method may be used.

The crosslinking reaction was performed in an alkaline aqueous solution with 1.0 M potassium hydroxide (KOH) at 20°C. CMCNa and HEC were dissolved in distilled water containing the desired amount of DVS. After mixing, the sample was left for 12-24 hours in order to obtain proper dissolution of the polymers. Potassium hydroxide was added, thereby starting the crosslinking reaction. All reactions were performed with a reaction solution having an overall polymer concentration equal to 2% by weight.

After 24 hours, the crosslinked hydrogel was broken zinto smaller pieces, by manually cutting the gel or by using an electric whisk, and was subsequently soaked in excess alcohol in order to reach an equilibrium. The solution surrounding the hydrogel was exchanged at least three times. Each time, an amount of alcohol corresponding to at least 5 times the weight of the hydrogel measured immediately after the crosslinking reaction was used. The soaking procedure was terminated after 36-48 hours. Subsequently, the swelled hydrogel was removed from the solution and desiccated.

After desiccation, the dried gels were ground using a laboratory mill. All subsequent measurements were performed on desiccated gel which had been treated in this manner.

### Test liquid

The test liquid which was used in the following test methods was synthetic urine, SUR. The composition of the SUR was 60 mmol/l KCl, 130 mmol/l NaCl, 3.5 mmol/l MgSO₄, 2.0 mmol/l CaSO₄•2H₂O, 300 mmol/l urea, 1 g/l of a 0.1% solution of Triton X-100 which is a surfactant sold by Aldrich.

### Free swelling capacity

Free swelling was determined by immersing a piece of dried gel in test liquid and allowing the gel to absorb liquid until saturated. The gel was subsequently removed from the liquid and weighed.

### Centrifuge retention capacity

The liquid retention after centrifugation for 10 minutes at 1500 rpm (corresponding to a load of approximately 300 g) was determined by weighing the gel before and after centrifugation.

### Liquid uptake capacity

The liquid uptake capacity for fibrous structures containing superabsorbent material was determined by letting test bodies made from fluffed cellulose pulp having 20% by weight of a crosslinked, dried, and ground gel absorb liquid according to a principle commonly known as "demand wettability". The measurements were made with a GATS-like apparatus. The test bodies were allowed to absorb liquid from a communicating vessel. The amount of liquid in the vessel was continuously measured using scales. The test continued for 2 hours, whereafter the amount of liquid which had been absorbed by the test body was recorded. By knowing the water uptake capacity of the fluffed cellulose pulp, the amount of liquid absorbed by the crosslinked gel could subsequently be calculated.

Each measurement was the mean value of two recordings made with identical test bodies.

### Example 1

Four samples consisting of crosslinked gel prepared from a 3:1 mixture of CMC and HEC were soaked in different solvents.

The solvents were:
i) methanol
ii) ethanol
iii) isopropanol
iv) water

Samples i)-iv) were air dried.

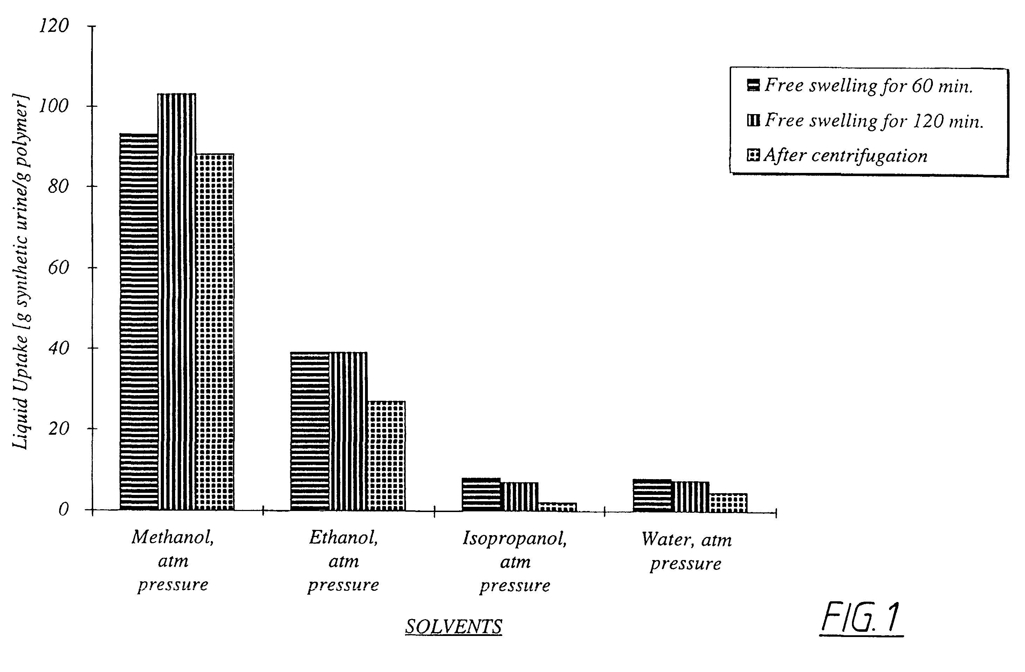

Liquid uptake was measured after free swelling for 60 minutes and 120 minutes. Liquid retention after centrifugation was also determined. As can be seen in Fig. 1, soaking in methanol produced a gel having excellent absorption characteristics.

### Example 2

The free swelling capacity was determined for desiccated gels comprising a mixture of HEC and CMC.

Five different ratios HEC/HEC+CMC were compared:
i) only CMC; HEC/HEC+CMC = 0
ii) CMC:HEC = 3:1; HEC/HEC+CMC = 0.25
iii) CMC:HEC = 1:1; HEC/HEC+CMC = 0.5
iv) CMC:HEC = 1:3; HEC/HEC+CMC = 0.75
v) only HEC; HEC/HEC+CMC = 1

The crosslinked gels were soaked in excess methanol, broken up into smaller pieces and air dried.

The free swelling capacity was determined after 60 minutes and after 120 minutes soaking in synthetic urine. Liquid retention after centrifugation was also measured.

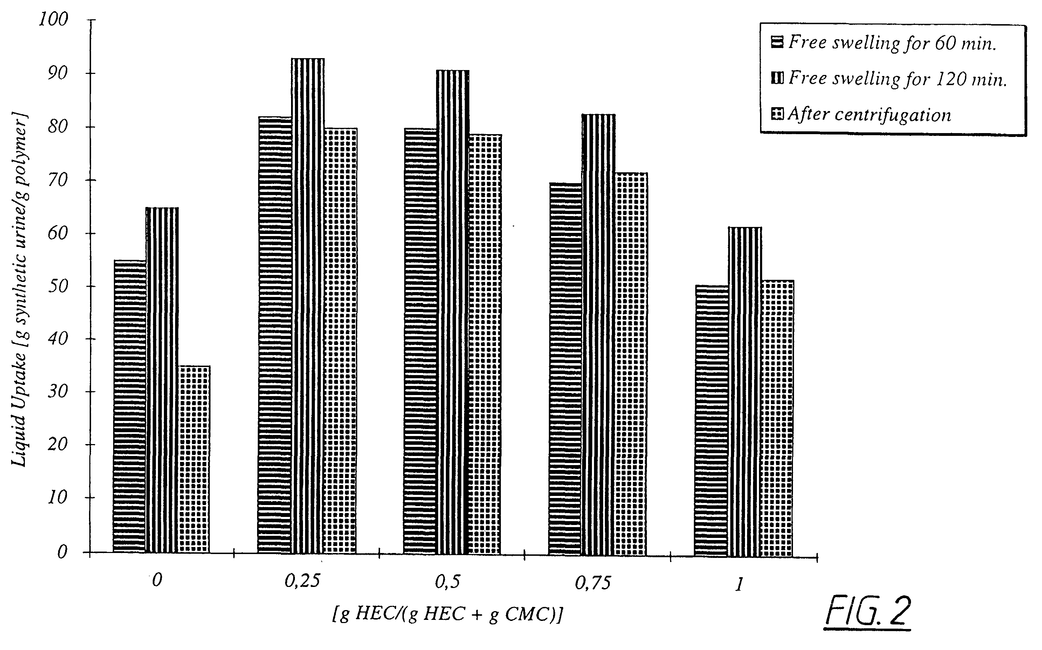

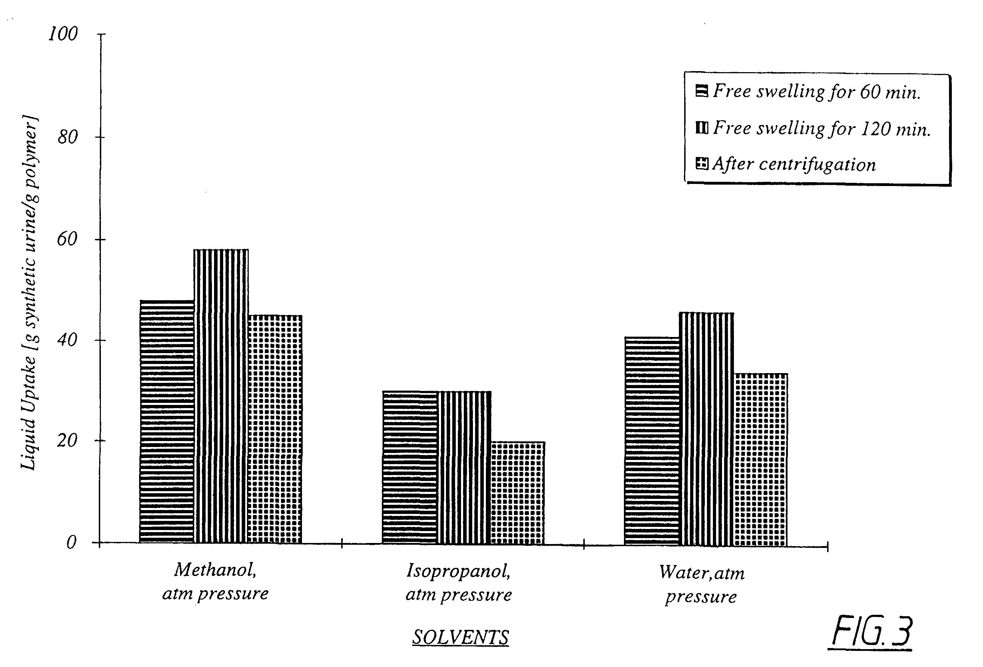

The results are shown in Fig. 2. All samples exhibit surprisingly high liquid uptake capacity. The ability to retain liquid after centrifugation is also very good.

### Example 3

Three samples consisting of crosslinked gel prepared from guar gum were soaked in different solvents.

The solvents were:
i) methanol
ii) isopropanol
iii) water

The samples were air dried after the solvent soaking step.

Liquid uptake was measured after free swelling for 60 minutes and 120 minutes. Liquid retention after centrifugation was also determined. The results are shown in Fig. 3. As for the CMC/HEC samples in Example 2, soaking in methanol produced a gel having considerably improved absorption characteristics.

### Example 4

The absorption capacity under compressive load was determined according to the liquid uptake capacity method for samples of a highly absorbent material produced in accordance with the invention. The absorption capacity was also measured for Sanwet IM7100 which is a commercially available polyacrylate-based absorption material produced by Clariant AG in Frankfurt, Germany and, further, for one type of CMC.

The amount of liquid absorbed by the different absorption materials measured in grams/gram absorption material appear in Table 1 below. It is evident that an absorption material which has been produced in accordance with the invention has a remarkably high absorption capacity, almost on a level with the absorption capacity of a polyacrylate-based absorption material and considerably higher than other biocompatible absorption materials.

**Table 1.**

| Sample | Pressure (kPa) | Tot. abs. (g) | Abs. cap. (g/g SAP) |
|---|---|---|---|
| Sample A | 0.57 | 26.1 | 39.3 |
| Sample B | 0.57 | 22.8 | 30.9 |
| IM7100 | 0.57 | 29.2 | 46.8 |
| Crosslinked CMC* | 0.57 | 23.2 | 31.9 |
| Sample A | 2.5 | 19.0 | 21.5 |
| Sample B | 2.5 | 19.1 | 21.6 |
| Crosslinked CMC* | 2.5 | 16.7 | 15.6 |

| | | | |
|---|---|---|---|
| Samples A and B = methanol treated CMC/HEC (3:1) samples * the preparation of superabsorbent based on crosslinked CMC is described, for instance, in EP-A-0 201 895 | | | |

## Claims

1. A method for the production of a highly absorbent polysaccharide-based absorption material, wherein a liquid solution containing a starting material in the form of a crosslinkable polysaccharide-based polymer is subjected to crosslinking to produce a liquid-swollen gel,
**characterized in that** the at least partially crosslinked gel is soaked in methanol or ethanol so that water is partially removed from the gel.

2. A method according to claim 1, wherein the at least partially crosslinked gel is soaked in methanol.

3. A method according to claim 1 or 2, wherein the solution containing the starting material is an aqueous solution.

4. A method according to claim 1, 2 or 3, wherein the crosslinking step is performed by adding a covalent crosslinking agent to the polymer solution.

5. A method according to any one of claims 1-4, wherein the starting material is a polymer blend comprising an electrically charged polysaccharide-based polymer and an electrically uncharged polysaccharide-based polymer.

6. A method according to claim 5, wherein the ratio between the charged polymer and the uncharged polymer is between about 2:1 and about 4:1 and preferably about 3:1.

7. A method according to claim 5 or 6, wherein the starting material is a mixture of carboxymethyl cellulose (CMC) and hydroxyethyl cellulose (HEC).

8. A method according to claim 1, 2 or 3, wherein the starting material comprises guar gum.

9. A polysaccharide-based, highly absorbent absorption material produced by crosslinking and drying a liquid solution containing a starting material in the form of a crosslinkable polysaccharide-based polymer, wherein the starting material, after crosslinking, exists in the form of a liquid-swollen gel, **characterized in that** the crosslinked, liquid-swollen hydrogel has been soaked in methanol or ethanol prior to drying of the gel.

10. A polysaccharide-based, highly absorbent absorption material according to claim 9, wherein the starting material is a polymer blend comprising an electrically charged polysaccharide-based polymer and an electrically uncharged polysaccharide-based polymer.

11. A polysaccharide-based, highly absorbent absorption material according to claim 10, wherein the ratio between the charged polymer and the uncharged polymer is between about 2:1 and about 4:1 and preferably about 3:1.

12. A polysaccharide-based, highly absorbent absorption material according to claim 10 or 11, wherein the starting material is a mixture of carboxymethyl cellulose (CMC) and hydroxyethyl cellulose (HEC).

13. A polysaccharide-based, highly absorbent absorption material according to claim 9, wherein the starting material comprises guar gum.

14. Absorbent article comprising the highly absorbent absorption material of any one of claims 9-13.

## Patentansprüche

1. Verfahren zur Herstellung eines stark absorbierenden Absorptionsmaterials auf Polysaccharidbasis, worin eine flüssige Lösung, die einen Ausgangsstoff in Form eines vernetzbaren Polymers auf Polysaccharidbasis enthält, einer Vernetzung unterzogen wird, um ein flüssigkeitsgequollenes Gel herzustellen, **dadurch gekennzeichnet, dass** das zumindest teilweise vernetzte Gel in Methanol oder Ethanol getaucht wird, so dass Wasser teilweise aus dem Gel entfernt wird.

2. Verfahren gemäss Anspruch 1, worin das zumindest teilweise vernetzte Gel in Methanol getaucht wird.

3. Verfahren gemäss Anspruch 1 oder 2, worin die Lösung, welche den Ausgangsstoff enthält, eine wässrige Lösung ist.

4. Verfahren gemäss Anspruch 1, 2 oder 3, worin der Vernetzungsschritt durchgeführt wird, indem man ein kovalentes Vernetzungsmittel zu der Polymerlösung gibt.

5. Verfahren gemäss einem der Ansprüche 1 bis 4, worin der Ausgangsstoff eine Polymermischung ist, die ein elektrisch geladenes Polymer auf Polysaccharidbasis und ein elektrisch nicht-geladenes Polymer auf Polysaccharidbasis umfasst.

6. Verfahren gemäss Anspruch 5, worin das Verhältnis zwischen dem geladenen Polymer und dem nicht-geladenen Polymer zwischen etwa 2:1 und etwa 4:1 und vorzugsweise etwa 3:1 beträgt.

7. Verfahren gemäss Anspruch 5 oder 6, worin der Ausgangsstoff eine Mischung aus Carboxymethylcellulose (CMC) und Hydroxyethylcellulose (HEC) ist.

8. Verfahren gemäss Anspruch 1, 2 oder 3, worin der Ausgangsstoff Guargummi umfasst.

9. Stark absorbierendes Absorptionsmaterial auf Polysaccharidbasis, das durch Vernetzen und Trocknen einer flüssigen Lösung erhalten wurde, die einen Ausgangsstoff in Form eines vernetzbaren Polymers auf Polysaccharidbasis enthält, worin der Ausgangsstoff nach dem Vernetzen in Form eines flüssigkeitsgequollenen Gels vorliegt, **dadurch gekennzeichnet, dass** das vernetzte flüssigkeitsgequollene Hydrogel vor dem Trocknen des Gels in Methanol oder Ethanol getaucht wurde.

10. Stark absorbierendes Absorptionsmaterial auf Polysaccharidbasis gemäss Anspruch 9, worin der Ausgangstoff eine Polymermischung ist, die ein elektrisch geladenes Polymer auf Polysaccharidbasis und ein elektrisch nicht-geladenes Polymer auf Polysaccharidbasis umfasst.

11. Stark absorbierendes Absorptionsmaterial auf Polysaccharidbasis gemäss Anspruch 10, worin das Verhältnis zwischen dem geladenen Polymer und dem nicht-geladenen Polymer zwischen etwa 2:1 und etwa 4:1 und vorzugsweise etwa 3:1 beträgt.

12. Stark absorbierendes Absorptionsmaterial auf Polysaccharidbasis gemäss Anspruch 10 oder 11, worin der Ausgangsstoff eine Mischung aus Carboxymethylcellulose (CMC) und Hydroxyethylcellulose (HEC) ist.

13. Stark absorbierendes Absorptionsmaterial auf Polysaccharidbasis gemäss Anspruch 9, worin der Ausgangsstoff Guargummi umfasst.

14. Absorbierender Gegenstand, welcher das stark absorbierende Absorptionsmaterial gemäss einem der Ansprüche 9 bis 13 umfasst.

## Revendications

1. Procédé de production d'un matériau hautement absorbant à base de polysaccharides, dans lequel on fait réticuler une solution d'un liquide contenant un produit de départ se présentant sous la forme d'un polymère réticulable à base de polysaccharide, de façon à obtenir un gel gonflé de liquide, et qui est **caractérisé en ce que** l'on fait tremper le gel, au moins partiellement réticulé, dans du méthanol ou dans de l'éthanol de manière à retirer partiellement l'eau du gel.

2. Procédé conforme à la revendication 1, dans lequel c'est dans du méthanol que l'on fait tremper le gel au moins partiellement réticulé.

3. Procédé conforme à la revendication 1 ou 2, dans lequel la solution contenant le produit de départ est une solution aqueuse.

4. Procédé conforme à la revendication 1, 2 ou 3, dans lequel on effectue l'étape de réticulation en ajoutant à la solution de polymère un agent de réticulation par liaisons covalentes.

5. Procédé conforme à l'une des revendications 1 à 4, dans lequel le produit de départ est un mélange de polymères comprenant un polymère à base de polysaccharide électriquement chargé et un polymère à base de polysaccharide non électriquement chargé.

6. Procédé conforme à la revendication 5, dans lequel le rapport entre le polymère chargé et le polymère non chargé vaut à peu près de 2/1 à 4/1, et de préférence environ 3/1.

7. Procédé conforme à la revendication 5 ou 6, dans lequel le produit de départ est un mélange de carboxyméthylcellulose (CMC) et d'hydroxyéthylcellulose (HEC).

8. Procédé conforme à la revendication 1, 2 ou 3, dans lequel le produit de départ contient de la gomme de guar.

9. Matériau hautement absorbant à base de polysaccharides, produit par réticulation et séchage d'une solution d'un liquide contenant un produit de départ se présentant sous la forme d'un polymère réticulable à base de polysaccharide, dans lequel le produit de départ se retrouve, après la réticulation, sous la forme d'un gel gonflé de liquide, et qui est **caractérisé en ce que** l'on a fait tremper dans du méthanol ou dans de l'éthanol l'hydrogel réticulé et gonflé de liquide, avant de faire sécher le gel.

10. Matériau hautement absorbant à base de polysaccharides, conforme à la revendication 9, dans lequel le produit de départ est un mélange de polymères comprenant un polymère à base de polysaccharide électriquement chargé et un polymère à base de polysaccharide non électriquement chargé.

11. Matériau hautement absorbant à base de polysaccharides, conforme à la revendication 10, dans lequel le rapport entre le polymère chargé et le polymère non chargé vaut à peu près de 2/1 à 4/1, et de préférence environ 3/1.

12. Matériau hautement absorbant à base de polysaccharides, conforme à la revendication 10 ou 11, dans lequel le produit de départ est un mélange de carboxyméthylcellulose (CMC) et d'hydroxyéthylcellulose (HEC).

13. Matériau hautement absorbant à base de polysaccharides, conforme à la revendication 9, dans lequel le produit de départ contient de la gomme de guar.

14. Article absorbant comprenant un matériau hautement absorbant conforme à l'une des revendications 9 à 13.
